# EUROPEAN PATENT APPLICATION

(11) **EP 4 480 456 A1**
(43) Date of publication of application: **25.12.2024**
(21) Application number: 23180298.4
(22) Date of filing: 20.06.2023
(51) Int. Cl.: A61F 2/46, A61F 2/08, A61F 2/30

(54) **IMPLANT, INSTRUMENT AND KIT FOR IMPLANTING AN IMPLANT**

(71) Applicant: Surgical Fusion Technologies GmbH, 8952 Schlieren (CH)
(72) Inventor: Mayer, Jörg, Niederlenz (CH); Müller, Andrea, Winterthur (CH); Vanoni, Michele, Zürich (CH)
(74) Representative: Vossius & Partner Patentanwälte Rechtsanwälte mbB

(57) **Abstract**

The present disclosure relates to an implant (10) configured to be at least partially inserted into at least one opening (44) in at least one bone (46) of a patient. The implant (10) includes at least one implant body (12) made at least partially of at least one resorbable material. The implant body (12) includes at least one first region (14) and at least one second region (16). The first region (14) has a plurality of open pores and the first region (14) has a greater porosity than the second region (16).

The present disclosure also relates to an instrument (42) for implanting an implant (10) into an opening (44) defined in at least one bone (46) of a patient.

## Description

Implants, e.g., bone implants, are used in a number of different fields and for a number of different purposes. For instance, implants are often used in reconstructive surgery, e.g., to repair damage caused by injuries, such as sports injuries. However, implants can be used for a number of further applications and/or in a number of further fields.

Implants, in particular bone implants, are often made from a resorbable material to assist in allowing the implant to be integrated into tissue, e.g., bone tissue, after the implant has been implanted. The process of integrating the bone implant into the bone tissue is often also referred to as osseointegration or bone bridging.

However, most, if not all, resorbable materials which are currently used in bone implants, e.g., calcium phosphate, have a relatively low strength, e.g., for transferring loads, forces, and/or moments to and/or from the bone. This may limit the number of potential applications of the respective bone implant and/or may result in failure of the implant and/or damage to the implant.

Moreover, at least some implants known from the prior art suffer from relatively slow osseointegration. For instance, many reconstructions, e.g., following sports injuries, suffer from relatively high revision rates due to relatively slow osseointegration. Further, in these early revision surgeries, the surgeons must maneuver around the pre-existing implants since the implant has not been replaced by bone yet. This limits the availability of the required good bone stock for the new fixation system.

However, the above-identified aspects have not, or at least not sufficiently, been addressed in the prior art. Thus, there is a need to improve the implants known from the prior art.

It is therefore an object of the present invention to provide an improved implant, in particular by providing improvement to one or more of the above-identified aspects.

The above-identified object is achieved by an implant according to a first aspect of the present disclosure, as defined by the features of claim 1. Preferred embodiments are defined by the features of the dependent claims, respectively.

Various exemplary embodiments of the present disclosure disclosed herein are directed to providing features that will become readily apparent by reference to the following description when taken in conjunction with the accompanying drawings. In accordance with various embodiments, exemplary devices are disclosed herein. It is understood, however, that these embodiments are presented by way of example and not limitation, and it will be apparent to those of ordinary skill in the art who read the present disclosure that various modifications to the disclosed embodiments can be made while remaining within the scope of the present disclosure.

Thus, the present disclosure is not limited to the exemplary embodiments and applications described and illustrated herein. Additionally, the specific order and/or hierarchy of steps in the methods disclosed herein are merely exemplary approaches. Based upon design preferences, the specific order or hierarchy of steps of the disclosed methods or processes can be re-arranged while remaining within the scope of the present disclosure. Thus, those of ordinary skill in the art will understand that the methods and techniques disclosed herein present various steps or acts in a sample order, and the present disclosure is not limited to the specific order or hierarchy presented unless expressly stated otherwise.

The implant may be configured to be at least partially inserted into at least one opening in at least one bone of a patient. The implant may include at least one implant body made at least partially of at least one resorbable material. Various resorbable materials are feasible, e.g., resorbable calcium phosphate, resorbable ceramic, resorbable glass, resorbable polymer, resorbable metal alloy, such as e.g., magnesium or zinc-based alloys. The implant may be at least partially made of at least one calcium phosphate ceramic. The implant may be made of a single resorbable material or a plurality of different resorbable materials.

The implant body may include at least one first region and at least one second region. The first region may have a plurality of open pores. The first region may have a greater porosity than the second region.

Porosity, more specifically a degree of porosity, within the meaning of the present disclosure, may be measured according to any known means/method, e.g., by means of micro-CT analysis, metallographic analysis, mercury porosimetry and/or according to ISO 15901-2:2022.

"Open pores" or "open porosity" is to be understood within the context of the present disclosure as relating to pores which are open to an environment, and optionally also with one another. The open pores may be open at at least a section or surface of the implant body which interfaces the bone, when the implant has been inserted into the opening of the bone. Thus, the open pores of the first region may facilitate osseointegration and/or bone bridging of the implant and the bone, e.g., by allowing and/or promoting bone ingrowth into the pores and/or by allowing and/or promoting a supply of nutrients and/or a vascular blood supply, at least partially into and/or through the implant body, e.g., to supply a graft which may be configured to be attached within a patient's body by the implant, with nutrients. This may accelerate osseointegration and/or bone bridging of the implant and the bone and reduce the risk of failure of the implant and/or revision rates. For instance, the implant may be configured to achieve osseointegration within 4 to 12 weeks and/or substantially full bone bridging within 12 months, preferably at a rate of higher than 0.5-1 mm per month, after implanting the implant into the patient. Osseointegration is generally understood as a formation of a direct interface or bony connection between the implant and the bone, whereas bone bridging refers to a building through or filling of a porous structure, i.e., of the implant, with bone.

The open pores of the first region may be provided by a surface roughness of the implant body along the first region(s). For instance, increasing the surface roughness of the implant body along the first region(s) may increase the size of the pores and/or the porosity in the first region(s).

Moreover, configuring the second region to have a lower porosity than the first region may provide the second region with a relatively dense structure to allow the second region to at least primarily provide stability and/or stiffness and/or strength to the implant. In particular, the open pores of the first region may cause the first region to be relatively brittle which may be compensated by the increased stability and and/or strength which is provided by the second region(s). In other words, the second region(s) may protect the relatively highly osteoconductive, porous volume of the first region(s) by providing a relatively stiff and/or rigid structure which may primarily bear loads acting on the implant. The second regions(s) may provide a framework which at least partially encases the first region(s).

In other words, the second region may primarily provide stability and/or strength to the implant, e.g., for load-bearing and/or load-transfer by the implant, i.e., to a greater extent than the first region, whereas the first region may facilitate the integration of the implant into the bone tissue due to the greater (open) porosity.

The second region(s) may provide a lattice-like support structure, i.e., a load-bearing framework, to the implant body to maintain implant integrity while providing a relatively quick osseointegration of the implant into the bone.

Though implants with open porosity are known, greater stability through the less porous second region may allow the open porosity along the first region(s) to be increased compared with implants known from the prior art.

Preferably, the second region has a relatively low porosity, e.g., as close to zero as possible. Alternatively, the second region may have some pores, e.g., open pores.

The implant may be configured to bear and/or transfer loads to and/or from the bone, e.g., as an anchor, such as a suture anchor configured to anchor one or more sutures and/or one or more tapes in bone for soft tissue fixation, a brace and/or reinforcement element to brace and/or reinforce one or more ligaments, a tethering device for configured to build tension bands, e.g., as to be used in the correction of spinal deformities, an interference screw, dowel or wedge for press fit based tendon or ligament fixation, an intramedullary fixation device, e.g., for metacarpal and metatarsal bones;, a osteotomy wedge and/or spacer for deformity corrections and arthrodesis, and a transarticular bar, dowel or pin for arthrodesis.

The first region is preferably mesoporous. Preferably, the first region has a porosity in a range of 5% by volume to 90% by volume, more preferably porosity in a range of 10% by volume to 90% by volume, more preferably in a range of 15% by volume to 90% by volume, more preferably in a range of 20% by volume to 90% by volume, more preferably in a range of 25% by volume to 90% by volume, more preferably in a range of 30% by volume to 90% by volume, more preferably in a range of 35% by volume to 90% by volume, more preferably in a range of 40% by volume to 90% by volume.

The porosity of the first region may be provided intrinsically by the resorbable material. For instance, calcium phosphate, intrinsically has a generally porous, preferably microporous/mesoporous, structure. The porosity, e.g., the distribution of the pores and/or the size of the pores, may be adjusted and/or customized, e.g., during forming of the implant body, e.g., during 3D printing, casting, and/or sintering of the implant body. The porosity may be provided, e.g., by drilling, in one or more certain regions of the implant body, preferably after the implant body has been manufactured, e.g., 3D printed or sintered. However, this may be less preferred.

The opening in the bone of the patient may have any shape and/or size, e.g., the opening may be elongated, short, etc. Preferably, the shape and/or size of the implant is configured to substantially match the shape and/or size of the opening. The opening may be a natural opening and/or a man-made opening, e.g., by drilling the opening into the bone.

The implant body may be made completely of a resorbable material. Alternatively, at least one coating of resorbable material may be provided on an outer surface of a core member of the implant body, the core member at least partially not being made from a resorbable material.

The implant may be configured to be used for a variety of different purpose and/or different applications. For instance, the implant may be used for reconstruction, e.g., following an injury, e.g., a sports injury. Preferably, the implant may be used to repair an at least partially torn ligament and/or tendon, e.g., an anterior cruciate ligament (ACL). However, a wide range of further purposes and/or applications of the implant are feasible, e.g., for conversion osteotomies, as connection/bridging elements for resected joints in arthrodesis, in arthrodesis systems, e.g., in foot and ankle, and/or for intramedullary fixation, e.g., in foot and ankle.

Although the present disclosure describes the invention primarily within the context of bone implants, the implant described herein may be used for insertion and fixation into at least one opening in a body of a patient in general, preferably into at least one opening in a tissue in a body of a patient. Thus, the present invention is not limited to bone implants.

The first region(s) and the second region(s) may be formed as a single integral piece. The implant body may be manufactured by using a variety of different manufacturing processes. For instance, the implant body may be manufactured by 3D printing, casting, sintering, etc.

Preferably, the plurality of pores of the first region each have a diameter in a range from 5 µm to 250 µm, preferably from 5 µm to 225 µm, more preferably from 5 µm to 200 µm, more preferably from 10 µm to 200 µm, more preferably from 15 µm to 200 µm, more preferably from 20 µm to 200 µm. Alternatively, the above-identified diameter values may be an average diameter of the plurality of pores of the first region.

Larger pores, e.g., macro pores, may generally improve a vascular blood supply at and/or at least partially into and/or through the implant body, whereas smaller pores, e.g., micro pores, may generally improve a supply of nutrients at and/or at least partially into the implant body. Hence, the dimension(s) of the pores may be selected and/or adjusted according to the desired and/or required functions. Providing a relatively broad range of pore sizes may ensure that at least both of the above-described functions can be provided.

Preferably, the implant body, preferably the first region of the implant body, has a total porosity, preferably a total open porosity, of at least 20%, preferably at least 30%, more preferably at least 40%, more preferably at least 50%, more preferably at least 60%, more preferably at least 70%, more preferably at least 80%.

Preferably, the implant is configured to:
connect the bone to at least one further structure of the patient's body, preferably at least one second bone;
   and/or
fixate at least one connective tissue and/or at least one connective tissue replacement graft to the bone, when the implant is at least partially inserted into the opening of the bone.

The connective tissue may be at least one ligament and/or at least one tendon and/or at least one graft. The graft may be artificially manufactured, e.g., to replace at least a section of a ligament, e.g., an anterior cruciate ligament (ACL).

Preferably, the implant is configured to transfer one or more loads:
between the bone and connective tissue and/or between the bone and at least one connective tissue replacement graft;
   and/or
between the bone and at least one further structure of the patient's body, preferably at least one second bone.

Preferably, the connective tissue is a ligament, preferably an anterior cruciate ligament, and/or a tendon. Alternatively, or additionally, the connective tissue replacement graft is a ligament graft and/or a tendon graft, preferably a graft for at least partially reconstructing an anterior cruciate ligament.

Preferably, the first region includes a plurality of channels defined at least partially in the first region. In other words, in addition to the plurality of open pores, the first region may include the plurality of channels. This may provide a bimodal porosity, i.e., regions with different degrees of porosity and/or types of porosity. At least some of the channels may penetrate at least some of the open pores of the first region(s). The channels may provide macro-porosity to the first region(s) within mesoporous areas of the first region(s).

The channels may promote and/or may accelerate osseointegration and/or bone bridging of the implant and the bone, e.g., by allowing perfusion, e.g., by means of blood, such as blood flowing from and/or flowing to blood vessels, through the channels. Alternatively, or additionally, the channels may, prior to implanting the implant and/or in an implanted state, may be configured to receive one or more active materials, such as bone marrow aspirate, stem cell/platelet enriched plasma, putty with growths factors, e.g., BMP2, BMP7 and/or TGF alpha. Hence, the channels may provide means for at least partially filling the implant with one or more active materials, such as those recited above.

At least some of the channels may be open to an environment and/or a hollow space of the implant body.

The channels may have any cross-sectional shape. For instance, the channels may have a substantially circular cross-sectional shape. However, alternatively, the channels may have a number of other cross-sectional shapes, e.g., polygonal, etc. The size and/or shape, e.g., the cross-sectional shape, of the channels may be configured and/or adapted to the respective application of the implant, e.g., according to one or more loads and/or one or more forces and/or one or more moments which is/are applied to or transferred by the implant, e.g., according to one or more directions and/or one or more values of one or more loads and/or one or more forces and/or one or more moments which is/are applied to or transferred by the implant. Preferably, the channels are configured to avoid or mitigate stress peaks within the implant, when the implant is being stressed.

At least some of the channels may have a uniform cross-sectional shape and/or uniform cross-sectional size along a direction of extension, e.g., along a longitudinal axis, of the channel(s). Alternatively, the cross-sectional shape and/or cross-sectional size of at least some of the channels may change and/or vary along a direction of extension, e.g., along a longitudinal axis, of the channel(s). For instance, at least some of the channels may extend conically through the implant. Preferably, the respective channels may have a larger diameter towards an outer surface of the implant than towards a center of the implant, e.g., towards a longitudinal axis of the implant body. In other words, the diameter of the respective channels may be reduced, preferably conically, in a direction towards a center of the implant body, e.g., from a diameter of 1 mm to a diameter of 300 µm.

Preferably, the channels have an open end which faces one or more walls of the bone, preferably one or more side walls of the bone, when the implant is inserted in the opening of the bone. In other words, the channels may be open at an end of the respective channel which faces one or more side walls of the bone, when the implant is inserted in the opening of the bone. This may facilitate perfusion, e.g., by means of blood, such as blood flowing from and/or flowing to blood vessels, through the channels to promote and/or accelerate osseointegration (or bone bridging) of the implant and the bone.

Preferably, the channels extend substantially radially and/or substantially perpendicularly to a longitudinal axis of the implant body at least partially through the implant body.

Preferably, the channels have a diameter of at least 100 µm, preferably at least 150 µm, more preferably at least 200 µm, more preferably at least 250 µm, more preferably at least 300 µm, more preferably at least 350 µm, more preferably at least 400 µm, more preferably at least 450 µm, more preferably at least 500 µm, more preferably at least 550 µm, more preferably at least 600 µm, more preferably at least 650 µm, more preferably at least 700 µm, more preferably at least 750 µm, more preferably at least 800 µm, more preferably at least 850 µm, more preferably at least 900 µm, more preferably at least 1 mm.

Preferably, the channels have a diameter of no more than 5 mm, preferably no more than 4.5 mm, more preferably no more than 4 mm, more preferably no more than 3.5 mm, more preferably no more than 3 mm, more preferably no more than 2.5 mm, more preferably no more than 2 mm, more preferably no more than 1.5 mm, more preferably no more than 1 mm, more preferably no more than 500 µm.

Preferably, the channels have a diameter in a range of 900 µm to 2 mm, preferably in a range of 800 µm to 2 mm, more preferably in a range of 700 µm to 2 mm, more preferably in a range of 600 µm to 2 mm, more preferably in a range of 500 µm to 2 mm, more preferably in a range of 400 µm to 2 mm.

If the channels have a non-circular cross-sectional shape, the above-identified diameter values and/or ranges are to be understood as referring to an equivalent diameter.

Preferably, the channels are arranged in an array which includes one or more rows and/or one or more columns of channels. This may facilitate perfusion, e.g., by means of blood, such as blood flowing from and/or flowing to blood vessels, through the channels across a relatively large section of the implant body to promote and/or accelerate osseointegration and/or bone bridging of the implant and the bone.

Preferably, the channels are arranged in at least two rows. Preferably, the channels of a first row of the at least two rows are arranged offset with respect to the channels of a second row of the at least two rows. In other words, the channels of the first row and the channels of the second row are arranged in a staggered manner. In other words, two adjacent rows preferably do not both have a channel in a cross-sectional segment which is perpendicular to a longitudinal axis of the implant body. This may further facilitate osseointegration.

Preferably, the second region has a porosity, preferably an open porosity, of less than 10%, preferably less than 5%, more preferably less than 1%. This may provide a relatively high degree of strength in the second region, in particular to compensate for a potentially reduced degree of strength in the first region due to the greater porosity in the first region.

Preferably, the implant further includes at least one securing device configured to interact with the bone, preferably by a form-fitting connection and/or a frictional connection, to fixate the implant to the bone. The securing device may provide a sturdy connection between the implant and the bone, at least until osseointegration or bone bridging between the implant and the bone has occurred. This may allow loads, forces, and/or moments which may act between the bone and the implant to be born and/or transmitted at least partially, preferably primarily, by the securing device, at least until osseointegration or bone bridging between the implant and the bone has occurred. In other words, the first region and the second region may not necessarily have to contribute, at least not significantly, to fixating the implant to the bone prior to osseointegration or bone bridging between the implant and the bone. This may prevent damage to the first region and second region and/or may allow the first region and second region to be configured more specifically to their intended purpose(s), e.g., to facilitate osseointegration between the implant and the bone (in the case of the first region) and provide strength and stability to the implant body (in the case of the second region). Thus, the securing device may provide an at least temporary fixation of the implant without the need for, at least initially, high implantation forces. Moreover, the securing device may provide fixation of the implant to the bone irrespective of bone density and may reduce or eliminate relative micro-motion between the implant and the bone, preferably even under cyclic loading. Preferably, the securing device is configured to attach to the bone without having to be screwed and/or rotated into the bone, e.g., via one or more threads. Instead, the securing device may be configured to be slid, or translationally moved, into at least a section of the bone. This may be advantageous since screwing the implant into the bone may cause damage, e.g., to the implant, the bone and/or one or more further components, e.g., ligaments, tendons, grafts, etc. Moreover, a thread, or other retention means, such as a barb, may generate stress peaks in the bone which may reduce the load-bearing capacity of the bone. Furthermore, the securing device may strengthen the bone, e.g., by infiltration of at least a portion of the securing device into the bone.

Preferably, the securing device is configured as an insert which is at least partially attached to the implant body. The securing device may be at least partially made of a relatively dense material, e.g., the securing device may have a greater density than the implant body. The securing device may be at least partially made of a polymer.

The securing device may be at least partially made of a resorbable polymer, e.g., a resorbable biocomposite, e.g., a composite of resorbable polymer and resorbable ceramic, and/or fiber composite, e.g., a fiber-reinforced biocomposite material.

Preferably, the securing device is made at least partially of a modifiable material, which is modifiable, preferably in a state in which the implant, or at least the securing device, is at least partially inserted in the opening of the bone, by adjusting at least one of at least one shape, at least one dimension, an aggregate state, a consistency, a plasticity, a viscosity and a hardness of the modifiable material. A fixation between the securing device and the bone may be achieved, or at least triggered, by modifying the modifiable material. In particular, modifying, e.g., liquifying, the modifiable material may trigger and/or change an interaction, e.g., a form-fitting connection and/or a frictional connection, between the modifiable material and the bone to fixate the implant to the bone, and optionally also between the modifiable material and connective tissue and/or at least one connective tissue replacement graft to fixate the connective tissue and/or at least one connective tissue replacement graft to the bone. For instance, modifying the modifiable material may cause the material of the securing device to at least partially infiltrate the bone.

Preferably, the securing device is made at least partially of an at least partially liquifiable material which is liquifiable in a state in which the implant, or at least the securing device, is at least partially inserted in the opening of the bone, preferably by applying energy, preferably ultrasound energy, to at least a portion of the securing device. The energy may be applicable directly, e.g., via direct contact between a source of energy and the securing device, and/or indirectly, e.g., via an additional energy transmitting device, to the securing device. The fixation via the liquifiable material may allow mechanical connecting elements, such as threads or barbs, which are conventionally used to fix an implant in a bone, to be omitted. The at least partially liquified liquifiable material may at least partially infiltrate the bone to at least partially engage with the bone.

Exemplary modifiable/ liquifiable materials and corresponding methods are described in WO 2011/054122 A1 and WO 02/069817 A1 which are herewith incorporated by reference in its entirety.

Preferably, the securing device is made at least partially of an at least partially curable and/or hardenable material which is curable and/or hardenable in a state in which the implant or at least the securing device, is inserted in the opening of the bone. The curable and/or hardenable material may be passively curable and/or hardenable, e.g., by stopping an application of energy to the securing device, e.g., by letting the securing device passively cool and harden and/or solidify. Additionally, or alternatively, the curable and/or hardenable material may be actively curable and/or hardenable, e.g., by actively cooling and/or applying pressure to the securing device.

Preferably, the implant includes a plurality of the at least one first region and a plurality of the at least one second region. Preferably, each second region is separated from an adjacent second region by at least one of the first regions. Preferably, the second regions are arranged in a network or framework manner, e.g., which at least partially encases the first regions.

Alternatively, the implant body may include a single first region which is configured as a base element of the implant body and a plurality of second regions or second region segments which are interconnected with and extend across one or more sections of the first region. Preferably, the second regions are arranged on an outer surface of the first region.

Preferably, all of the second regions are interconnected. This may provide a sturdier construction and/or a better force distribution within the implant body. Preferably, each of the first regions is completely separated from an adjacent first region of the first regions. Preferably, each of the first regions is arranged between adjacent portions of the second regions.

Preferably, at least some of the first regions and/or at least some of the second regions extend axially along the implant body. Preferably, at least some of the second regions extend axially along the entire length of the implant body. Preferably, at least some of the second regions are not interrupted by any first region(s) axially along the implant body.

Preferably, the implant further includes at least one attachment means for attaching connective tissue and/or a connective tissue replacement graft to the implant, preferably the implant body.

Preferably, the attachment means includes at least one groove which is defined in the implant body. Preferably, the groove is configured to at least partially receive the connective tissue and/or the connective tissue replacement graft. Preferably, the groove extends at least partially along a longitudinal axis of the implant body.

Preferably, the groove is open towards at least one side of the implant body.

Preferably, the securing device is arranged along at least a section of the groove.

Preferably, the implant includes a distal end section which terminates in a leading edge, wherein the distal end section is tapered towards the leading edge.

Preferably, the implant includes at least one adapter made of a different material than the implant body. The adapter may be configured to be engaged by an instrument, e.g., an instrument according to any of the embodiments described below, for implanting the implant. Hence, the adapter may be provided as in intermediate member between instrument and the main body of the implant. In other words, the adapter may provide an interface between the instrument and the main body. This may allow the adapter to be configured to facilitate an attachment of the implant to the instrument, i.e., without potentially causing adverse effects to the main body of the implant body. This may allow the configuration of the main body to be targeted more specifically to osteointegration and/or bone bridging between the implant and the bone, whereas the function of the connecting the implant to the instrument may be provided by the adapter.

The adapter may be fixed attached to the main body, preferably such that the adapter remains attached to the main body, when the implant has been released by the instrument.

Preferably, the adapter is adhesively bonded to the main body. The adhesive bond may include an intermediate material between the adapter and the main body. The adapter may be bonded to the main body by means of a liquifiable material, i.e., a material which can be liquified such that the liquified material engages with the adapter and the main body, whereupon the liquified material solidifies to fixedly connect the adapter and the main body. The same principle and/or the same material as the material described above for connecting the implant with the bone may be employed for attaching the adapter to the main body. Alternatively, the adapter and the main body may be adhesively bonded without any additional material therebetween, e.g., by allowing cross-linking between the material of the main body and the material of the adapter.

Preferably, the adapter is made of a biocompatible material. For instance, the adapter may be made of Polyether ether ketone (PEEK) and/or polylactic acid (PLA).

The adapter may also be configured to absorb one or more loads and/or shocks between the instrument and the implant. In other words, the adapter may be configured to absorb, and preferably dampen, any force, loads, and/or shocks which may occur between the instrument and the implant. This may be achieved by manufacturing the adapter from a suitable material, e.g., a material which is more compressible and/or softer and/or has a greater degree of internal damping than an adjacent material of the instrument and/or the implant, and/or by configuring the shape of the adapter accordingly, e.g., as a spring-like element.

The object mentioned at the beginning is also solved by a method for implanting at least one implant, preferably at least one implant according to any of the embodiments disclosed herein, in at least one opening in at least one bone of a patient. The features, embodiments, and advantages described above with respect to the implant apply to the method accordingly.

The method may include:
(a) providing the implant which includes at least one implant body made at least partially of at least one resorbable material, wherein the implant body may include at least one first region and at least one second region, wherein the first region may have a plurality of open pores; and wherein the first region may have a greater porosity than the second region.

The method may further include:
(b) inserting the implant at least partially into the opening in the bone.

The method may further include:
(c) fixating the implant to the bone.

Preferably, at least step (c) includes at least partially modifying, preferably at least partially liquifying, a modifiable material, preferably a liquifiable material, which is at least partially attached to the implant body, preferably in a state in which the implant, or at least the modifiable material, is at least partially inserted in the opening of the bone, by adjusting at least one of at least one shape, at least one dimension, an aggregate state, a consistency, a plasticity, a viscosity and a hardness of the modifiable material.

Preferably, the modifiable material is at least partially modified by applying energy, preferably ultrasound energy, to at least a portion of the modifiable material. Exemplary modifiable/ liquifiable materials which are modified by applying energy preferably ultrasound energy, and corresponding methods are described in WO 2011/054122 A1 and WO 02/069817 A1 which is are herewith incorporated by reference in its entirety.

Preferably, at least step (c) includes at least partially curing an at least partially curable material which is at least partially attached to the implant body, in a state in which the implant, or at least the curable material, is inserted in the opening of the bone.

The object mentioned at the beginning is also solved by a method for fixating at least one connective tissue and/or at least one connective tissue replacement graft to at least one bone. The features, embodiments, and advantages described above with respect to the implant apply to the method accordingly.

The method may include:
(a) providing at least one implant, preferably at least one implant according to any of the embodiments disclosed herein, which includes at least one implant body made at least partially of at least one resorbable material, wherein the implant body may include at least one first region and at least one second region, wherein the first region may have a plurality of open pores; and wherein the first region may have a greater porosity than the second region.

The method may further include:
(b) inserting the implant at least partially into at least one opening in the bone.

The method may further include:
(c) operatively connecting the connective tissue and/or at least one connective tissue replacement graft with the implant.

The method may further include:
(d) fixating the implant to the bone.

Moreover, the instruments known from the prior art for inserting the respective implant into the respective bone have several drawbacks. For instance, the instruments may only work relatively well with certain implants, e.g., implants made of a certain material. For instance, many instruments rely on a screw connection between the instrument and the implant for attaching the implant to the instrument. However, for some implants, e.g., implants made from certain materials, e.g., a more porous material, such a connection may be disadvantageous, e.g., by providing a relatively weak connection between the implant and the instrument which may fail.

Furthermore, at least some of the instruments known from the prior art have a release mechanism for releasing the implant from the implant which may be disadvantageous, e.g., since the release mechanism is time-consuming, tedious, complicated and/or may have one or more adverse effects on the implant and/or the site of application in the patient's body, e.g., surrounding tissue.

In particular, the instruments known from the prior art for inserting the respective implant into the bone may have several drawbacks when being combined with the implant according to any of the embodiments described herein.

It is therefore an object of the present invention to provide an improved instrument, in particular by providing improvement to one or more of the above-identified aspects.

The above-identified object is achieved by an instrument according to a further aspect of the present disclosure.

The instrument is configured for implanting an implant, preferably an implant according to any of the embodiments described herein, into the at least one opening defined in at least one bone of a patient.

Although the instrument may primarily be described herein in relation to the implant according to the present invention, the instrument may alternatively be employable with a range of other implants which are not described herein. Hence, the instrument according to the present invention may be employable independently from the implant according to the present invention.

Preferably, the instrument is configured such that the implant is attachable to and/or detachable from the instrument by translationally displacing the instrument relative to the implant and engaging the instrument, preferably a distal end of the instrument, with the implant. In other words, the instrument may be attached to the implant by merely pushing the instrument against the implant and/or vice versa, thereby actuating or activating one or more engagement mechanisms for fixedly attaching the implant to the instrument. To detach the instrument from the implant, the instrument may merely be pulled, e.g., proximally, from the implant, when the implant has been implanted within the patient's body, to release the one or more engagement mechanisms.

In other words, the instrument may be configured such that the implant is attachable to and/or detachable from the instrument without screwing/rotating the instrument and/or the implant, e.g., about a longitudinal axis of the instrument and/or a longitudinal axis of the implant. Thus, the instrument and the implant do not have to be provided with a screw thread for attaching the implant to the instrument. As described above, many instruments rely on a screw connection between the instrument and the implant for attaching the implant to the instrument. However, for some implants, e.g., implants made from certain materials, such a connection may be disadvantageous, e.g., by providing a relatively weak connection between the implant and the instrument which may fail. Thus, omitting such a screw mechanism may allow the implant to be used with a wider range of implants, in particular implants made of different materially which are less suitable for screw connections, e.g., implants made of a material which has a greater degree of porosity and/or which is more brittle than other materials. Moreover, rotating the instrument and/or the implant to unscrew the instrument from the implant, once the implant has been implanted inside the patient's body, may have one or more adverse effects on the site of application within the patient's body, in particular on surrounding tissue e.g., with respect to a graft which the implant is intended/configured to attach to the bone, which can be avoided by the configuration described above. Instead of a screw connection, the attachment of the implant to the instrument may be provided by a variety of different connection configurations, snap-fit, a vacuum, a frictional connection, etc., some of which are described below.

Alternatively, the instrument may include at least one screw thread configured to engage at least one screw thread of the implant, wherein the screw thread of the implant may be defined in or on an adapter configured to be arranged between a main body of the implant and the instrument. This may provide a screw connection for attaching the implant to the instrument, while reducing potential adverse effects on the implant, e.g., by manufacturing the main body of the implant from a relatively porous material and manufacturing the adapter from a different material, e.g., a less porous and/or more sturdy material.

The instrument may have at least one channel for introducing at least one securing device, e.g., a modifiable/liquifiable material, towards the implant. The channel may also be used to introduce an application device for applying energy, e.g., thermal energy and/or ultrasound energy, to the securing device to at least partially modify/liquify the securing device. The instrument may have at least one channel, which may be the same channel for introducing at least one securing device mentioned above or may be a further (different) channel, for introducing at least one fixating device, e.g., at least one k-wire, into, preferably completely through, the instrument. The fixating device may be employed to temporarily fixate the instrument and/or the implant and/or a graft to the bone and/or to guide the implant towards the target location of the implant within the patient's body, e.g., in a minimally invasive procedure.

Alternatively, the instrument may be configured for use with implants in which the securing device is pre-mounted, e.g., in a cavity defined within the implant, prior to inserting the implant into the opening of the bone. In this case, the channel described above may also be provided in the instrument, e.g., to introduce an application device for applying energy to the securing device.

Preferably, the instrument includes at least one retention device configured to engage with and/or be received in at least one undercut defined in at least a section of the implant to releasably fix the implant to the instrument. Preferably, the retention device is arranged at a distal end of the instrument. The undercut may be configured as a slot which extends partially or completely, preferably circumferentially, about the implant. The undercut may be provided along at least a portion of an outer circumference of the implant. Alternatively, or additionally, an undercut may be defined in a cavity of the implant, e.g., in at least one channel of the implant. The channel may be provided in the implant for one or more other functions, e.g., for introducing a modifiable/liquifiable material for attaching the implant to the bone into or to the implant. Hence, the cavity, e.g., channel, already present in the implant for other reasons may be utilized for attaching the implant to the instrument.

The undercut may include a ramp which extends radially outward in a proximal direction. The ramp may facilitate engagement and/or disengagement of the retention device from the implant.

Preferably, the retention device is at least partially deflectable, preferably in a direction which is substantially perpendicular to a longitudinal axis of the instrument and/or a longitudinal axis of the implant, such that the retention device is at least partially deflected, when the implant is being attached to and/or detached from the instrument. In other words, the retention device may extend from a main section of the instrument and may be deflectable relative to the main section. The deflectability of the retention device may be provided by a suitable material, e.g., a flexible material such as an elastomer, of the retention device, and/or by the dimension(s) and/or shape of at least a deflectable portion of the retention device, e.g., by providing one or more (deflectable) sections of the retention device with a reduced thickness. For instance, the retention device may be configured as one or more deflectable arms which extend distally from a main section of the instrument. The retention device may be configured to engage with the implant via a snap fit.

The retention device may be at least partially made of a material, or may include an element made of a material, which is configured to absorb one or more loads and/or shocks between the instrument and the implant.

Preferably, an outer measurement of the instrument at an interface between the instrument and the implant and an outer measurement of the implant at the interface are substantially identical.

Preferably, the instrument includes at least one protective member arranged at least partially at an interface between the instrument and the implant. The protective member may be configured to absorb one or more loads and/or shocks between the instrument and the implant. In other words, the protective member may be configured to absorb, and preferably dampen, any force, loads, and/or shocks which may occur between the instrument and the implant. This may be achieved by manufacturing the protective member from a suitable material, e.g., a material which is more compressible and/or softer and/or has a greater degree of internal damping than an adjacent material of the instrument and/or the implant, and/or by configuring the shape of the protective member accordingly, e.g., as a spring-like element. The retention device described above may include the protective member(s).

The protective member may be integrated into and/or attached to the instrument and/or the implant, e.g., in a cavity or groove thereof. The protective member may be configured to remain attached to the implant, e.g., the main body of the implant, when the implant has been released by the instrument.

Preferably, the instrument includes at least one sheath which is at least partially retractable relative to the implant, preferably in a proximal direction. Preferably, the sheath is configured to at least partially cover, preferably circumferentially, preferably along a longitudinal axis of the implant and/or a longitudinal axis of the instrument, the retention device and/or the implant, preferably an outer surface thereof, at least in a first position of the sheath. The sheath may be configured to be at least partially retracted from the first position to a second position, preferably to at least partially uncover or further uncover the retention device and/or the implant. The sheath, when in the first position, may prevent the implant from releasing from the instrument, or at least may reduce the risk thereof. For instance, the sheath may be configured to exert at least one force, preferably at least one force which is directed radially inward, onto the implant, e.g., directly, i.e., via direct contact between the sheath and the implant, and/or indirectly, e.g., via at least one intermediate member between the sheath and implant, e.g., the retention device described above. Retracting the sheath to the second position may allow, urge, and/or facilitate the release of the implant from the instrument, e.g., when the implant has been implanted in the patient's body. For instance, retracting the sheath may allow the retention device to be released from the implant. The instrument may be configured such that the retraction of the sheath may be performed manually by a user or by an integrated mechanism of the instrument, such as by one or more actuators, e.g., after receiving a user input, such as by pushing a button.

Alternatively, or additionally, the sheath may be configured to exert at least one force, preferably at least one force which is directed radially outward, onto the bone. Since a portion of the bone, e.g., the spongy bone tissue, is relatively soft and has a relatively low degree of creep, in particular during the relatively short application time in which the instrument is used on the patient to insert the implant, the force exerted by the sheath onto the bone may elastically urge/push the surrounding or adjacent bone tissue away, preferably radially outward, from the implant. Retracting the sheath proximally may allow the bone tissue to at least partially return to its original state, i.e., towards the implant, e.g., due to the relatively low degree of creep of the spongy bone tissue. The implant may be sized and/or shaped such that, when the sheath has been retracted, the bone tissue engages with and presses again the implant, to effectively form a frictional/press fit between the bone and the implant to substantially hold the implant in place.

Preferably, the instrument includes at least one expandable member configured to be inserted into at least one cavity of the implant. The expandable member may be configured to expand once the expandable member has been inserted in the cavity. The expandable member may be configured as a spreading member. The expandable member may be configured to actively and/or passively expand, once the expandable member has been inserted in the cavity. For, instance, the expandable member may include at least at least one biased element, e.g., at least one biased arm, preferably a plurality of biased elements/arms, which are biased in a biasing direction, e.g., outwards. The biased element(s) may be deflected via at least one deflecting force, which may be exerted manually by a user and/or via interaction with a surface of the implant, in a deflecting direction opposite to the biasing direction to allow insertion of the expandable member into the cavity of the implant. Once the expandable member has been inserted in the cavity, i.e., once the deflecting force has been reduced or eliminated, the biased element(s) may at least partially expand in the biasing direction to lock, preferably releasably lock, the expandable member in place in the cavity of the implant.

Preferably, the instrument includes at least one tensioning member configured to exert at least one force onto the implant in a proximal direction. This may provide (axial) tension between the instrument and the implant, e.g., to maintain the implant in a substantially stationary position relative to the instrument. In other words, this may reduce potential movement, in particular axial movement, of the implant relative to the instrument. This may increase the accuracy and/or efficiency of the implanting procedure of the implant into the patient's body. The tensioning member may be substantially flexible. For instance, the tensioning member may be configured as a band, preferably a stretchable band, or thread. Alternatively, the tensioning member may be a substantially rigid member, such as a rod.

Preferably, the instrument is configured to generate a vacuum to fix the implant to the instrument. For instance, the instrument may include at least one vacuum generating mechanism, e.g., a pump, forgenerating the vacuum. The vacuum generating mechanism may be manually driven, e.g., as a hand-driven pump, and/or may be electrically and/or pneumatically powered.

The present invention also relates to a kit including at least one implant, preferably at least one implant according to any of the embodiments described herein, and at least one instrument, preferably at least one instrument according to any of the embodiments described herein.

Preferably, the kit further includes at least one adapter made of a different material than the implant body. The adapter may be configured to be engaged by an instrument for implanting the implant.

The following list of aspects provides alternative and/or further features of the invention:
1. An implant configured to be at least partially inserted into at least one opening in a body of a patient, preferably into at least one opening in a tissue in a body of a patient, preferably into at least one opening in at least one bone of a patient, the implant including:
   at least one implant body which is preferably made at least partially of at least one resorbable material;
   wherein the implant body includes at least one first region and at least one second region,
   wherein the first region has a plurality of pores, preferably a plurality of open pores; and
   wherein the first region has a greater porosity than the second region.
2. The implant according to aspect 1, wherein the plurality of pores of the first region each have a diameter in a range from 5 µm to 250 µm, preferably from 5 µm to 225 µm, more preferably from 5 µm to 200 µm, more preferably from 10 µm to 200 µm, more preferably from 15 µm to 200 µm, more preferably from 20 µm to 200 µm.
3. The implant according to aspect 1 or 2, wherein the implant body, preferably the first region of the implant body, has a total porosity, preferably a total open porosity, of at least 20%, preferably at least 30%, more preferably at least 40%, more preferably at least 50%, more preferably at least 60%, more preferably at least 70%, more preferably at least 80%.
4. The implant according to any of the preceding aspects, wherein the implant is configured to:
   connect the bone to at least one further structure of the patient's body, preferably at least one second bone;
      and/or
   fixate at least one connective tissue and/or at least one connective tissue replacement graft to the bone, when the implant is at least partially inserted into the opening of the bone.
5. The implant according to any of the preceding aspects, wherein the implant is configured to transfer one or more loads:
   between the bone and connective tissue and/or between the bone and at least one connective tissue replacement graft;
      and/or
   between the bone and at least one further structure of the patient's body, preferably at least one second bone.
6. The implant according to aspect 4 or 5, wherein:
   the connective tissue is a ligament, preferably an anterior cruciate ligament, and/or a tendon;
      and/or
   the connective tissue replacement graft is a ligament graft and/or a tendon graft, preferably a graft for at least partially reconstructing an anterior cruciate ligament.
7. The implant according to any of the preceding aspects, wherein the first region includes a plurality of channels defined at least partially in the first region.
8. The implant according to aspect 7, wherein the channels have an open end which faces one or more walls which at least partially define the opening, preferably one of more walls of the bone, preferably one or more side walls of the bone, when the implant is inserted in the opening.
9. The implant according to aspect 7 or 8, wherein the channels extend substantially radially and/or substantially perpendicularly to a longitudinal axis of the implant body at least partially through the implant body.
10. The implant according to any of aspects 7 to 9, wherein the channels have a diameter of at least 100 µm, preferably at least 150 µm, more preferably at least 200 µm, more preferably at least 250 µm, more preferably at least 300 µm, more preferably at least 350 µm, more preferably at least 400 µm, more preferably at least 450 µm, more preferably at least 500 µm, more preferably at least 550 µm, more preferably at least 600 µm, more preferably at least 650 µm, more preferably at least 700 µm, more preferably at least 750 µm, more preferably at least 800 µm, more preferably at least 850 µm, more preferably at least 900 µm, more preferably at least 1 mm.
11. The implant according to any of aspects 7 to 10, wherein the channels have a diameter of no more than 5 mm, preferably no more than 4.5 mm, more preferably no more than 4 mm, more preferably no more than 3.5 mm, more preferably no more than 3 mm, more preferably no more than 2.5 mm, more preferably no more than 2 mm, more preferably no more than 1.5 mm, more preferably no more than 1 mm, more preferably no more than 500 µm.
12. The implant according to any of aspects 7 to 11, wherein the channels have a diameter in a range of 900 µm to 2 mm, preferably in a range of 800 µm to 2 mm, more preferably in a range of 700 µm to 2 mm, more preferably in a range of 600 µm to 2 mm, more preferably in a range of 500 µm to 2 mm, more preferably in a range of 400 µm to 2 mm.
13. The implant according to any of aspects 7 to 12, wherein the channels are arranged in an array which includes one or more rows and/or one or more columns of channels.
14. The implant according to any of aspects 7 to 13, wherein the channels are arranged in at least two rows, wherein the channels of a first row of the at least two rows are arranged offset with respect to the channels of a second row of the at least two rows.
15. The implant according to any of the preceding aspects, wherein the second region has a porosity, preferably an open porosity, of less than 10%, preferably less than 5%, more preferably less than 1%.
16. The implant according to any of the preceding aspects, further including at least one securing device configured to interact with one or more walls which at least partially define the opening, preferably to interact with the bone, preferably by a form-fitting connection and/or a frictional connection, to fixate the implant, preferably to the bone.
17. The implant according to aspect 16, wherein the securing device is configured as an insert which is at least partially attached to the implant body.
18. The implant according to aspect 16 or 17, wherein the securing device is made at least partially of a modifiable material, which is modifiable, preferably in a state in which the implant, or at least the securing device, is at least partially inserted in the opening, by adjusting at least one of at least one shape, at least one dimension, an aggregate state, a consistency, a plasticity, a viscosity and a hardness of the modifiable material.
19. The implant according to any of aspects 16 to 18, wherein the securing device is made at least partially of an at least partially liquifiable material which is liquifiable in a state in which the implant, or at least the securing device, is at least partially inserted in the opening, preferably by applying energy, preferably ultrasound energy, to at least a portion of the securing device.
20. The implant according to any of aspects 16 to 19, wherein the securing device is made at least partially of an at least partially curable material which is curable in a state in which the implant or at least the securing device, is inserted in the opening.
21. The implant according to any of the preceding aspects, including a plurality of the at least one first region and a plurality of the at least one second region, wherein each second region is separated from an adjacent second region by at least one of the first regions.
22. The implant according to aspect 21, wherein at least some of the first regions and/or at least some of the second regions extend axially along the implant body.
23. The implant according to any of the preceding aspects, further including at least one attachment means for attaching connective tissue and/or a connective tissue replacement graft to the implant, preferably the implant body.
24. The implant according to aspect 23, wherein the attachment means includes at least one groove which is defined in the implant body and configured to at least partially receive the connective tissue and/or the connective tissue replacement graft, preferably wherein the groove extends at least partially along a longitudinal axis of the implant body.
25. The implant according to aspect 24, wherein the groove is open towards at least one side of the implant body.
26. The implant according to aspect 24 or 25, when dependent from any of aspects 16 to 20, wherein the securing device is arranged along at least a section of the groove.
27. The implant according to any of the preceding aspects, wherein the implant includes a distal end section which terminates in a leading edge, wherein the distal end section is tapered towards the leading edge.
28. The implant according to any of the preceding aspects, further including at least one adapter made at least partially of a different material than the implant body, preferably wherein the adapter is configured to be engaged by an instrument for implanting the implant.
29. The implant according to aspect 28, wherein the adapter is bonded, preferably adhesively bonded, to the main body.
30. The implant according to aspect 28 or 29, wherein the adapter is made of a biocompatible material.
31. An instrument for implanting an implant, preferably an implant according to any of the preceding aspects, into at least one opening defined in a body of a patient, preferably into at least one opening in a tissue in a body of a patient, preferably into at least one opening in at least one bone of a patient.
32. The instrument according to aspect 31, wherein the instrument is configured such that the implant is attachable to and/or detachable from the instrument by translationally displacing the instrument relative to the implant and engaging the instrument, preferably a distal end of the instrument, with the implant.
33. The instrument according to aspect 31 or 32, wherein the instrument includes at least one retention device configured to engage with and/or be received in at least one undercut defined in at least a section of the implant to releasably fix the implant to the instrument, preferably wherein the retention device is arranged at a distal end of the instrument.
34. The instrument according to aspect 33, wherein the retention device is at least partially deflectable, preferably in a direction which is substantially perpendicular to a longitudinal axis of the instrument and/or a longitudinal axis of the implant, such that the retention device is at least partially deflected, when the implant is being attached to and/or detached from the instrument.
35. The instrument according to any of aspects 31 to 34, wherein the instrument includes at least one protective member arranged at least partially at an interface between the instrument and the implant, wherein the protective member is configured to absorb one or more loads and/or shocks between the instrument and the implant.
36. The instrument according to any of aspects 31 to 35, wherein the instrument includes at least one sheath which is at least partially retractable relative to the implant, preferably in a proximal direction, preferably wherein the sheath is configured to at least partially circumferentially cover the retention device and/or the implant at least in a first position of the sheath and the sheath is configured to be at least partially retracted from the first position to a second position to at least partially uncover or further uncover the retention device and/or the implant.
37. The instrument according to any of aspects 31 to 36, wherein the instrument includes at least one expandable member configured to be inserted into at least one cavity of the implant, wherein the expandable member is configured to expand once the expandable member has been inserted in the cavity.
38. The instrument according to any of aspects 31 to 37, wherein the instrument includes at least one tensioning member configured to exert at least one force onto the implant in a proximal direction.
39. The instrument according to any of aspects 31 to 38, wherein the instrument is configured to generate a vacuum to fix the implant to the instrument.
40. A kit including at least one implant, preferably at least one implant according to any of aspects 1 to 30, and at least one instrument, preferably at least one instrument according to any of aspects 31 to 39.
41. The kit according to aspect 40, further including at least one adapter made at least partially of a different material than the implant body, preferably wherein the adapter is configured to be engaged by an instrument for implanting the implant.
42. A method for implanting at least one implant, preferably at least one implant according to any of aspects 1 to 30, into at least one opening in a body of a patient, preferably into at least one opening in a tissue in a body of a patient, preferably into at least one opening in at least one bone of a patient, preferably using an instrument according to any of aspects 31 to 39, the method including:
   (a) providing the implant which includes at least one implant body which is preferably made at least partially of at least one resorbable material, wherein the implant body includes at least one first region and at least one second region, wherein the first region has a plurality of pores, preferably a plurality of open pores; and wherein the first region has a greater porosity than the second region;
   (b) inserting the implant at least partially into the opening, preferably in the bone; and
   (c) fixating the implant, preferably to the bone.
43. The method according to aspect 42, wherein at least step (c) includes at least partially modifying, preferably at least partially liquifying, a modifiable material, preferably a liquifiable material, which is at least partially attached to the implant body, preferably in a state in which the implant, or at least the modifiable material, is at least partially inserted in the opening, by adjusting at least one of at least one shape, at least one dimension, an aggregate state, a consistency, a plasticity, a viscosity and a hardness of the modifiable material.
44. The method according to aspect 43, wherein the modifiable material is at least partially modified by applying energy, preferably ultrasound energy, to at least a portion of the modifiable material.
45. The method according to any of aspects 42 to 44, wherein at least step (c) includes at least partially curing an at least partially curable material which is at least partially attached to the implant body, in a state in which the implant, or at least the curable material, is inserted in the opening.
46. A method for fixating at least one connective tissue and/or at least one connective tissue replacement graft to at least one bone, preferably using an instrument according to any of aspects 31 to 39, the method including:
   (a) providing at least one implant, preferably at least one implant according to any of aspects 1 to 30, which includes at least one implant body which is preferably made at least partially of at least one resorbable material, wherein the implant body includes at least one first region and at least one second region, wherein the first region has a plurality of pores, preferably a plurality of open pores; and wherein the first region has a greater porosity than the second region;
   (b) inserting the implant at least partially into at least one opening in the bone;
   (c) operatively connecting the connective tissue and/or at least one connective tissue replacement graft with the implant; and
   (d) fixating the implant to the bone.

Embodiments of the present invention are further elucidated below with reference to the figures. The figures are schematic drawings and as such may not show all details of the systems and their components. Particularly, the drawings are not necessarily to scale and the shown dimensions are only exemplary and may vary. The drawings illustrate exemplary embodiments to provide a thorough understanding of the present invention. The drawings are not intended to limit the scope of the invention, which is defined by the appended claims and is to include the equivalents thereof.
- Fig. 1: shows, in a schematic perspective view, an implant according to an embodiment of the present invention;
- Fig. 2: shows, in a schematic cross-sectional view, an implant according to a further embodiment of the present invention;
- Fig. 3: shows, in a schematic cross-sectional view, an implanting procedure for implanting the implant shown in Figs. 1 and 2;
- Fig. 4: further shows, in a schematic cross-sectional view, an implanting procedure for implanting the implant shown in Figs. 1 and 2;
- Fig. 5: further shows, in a schematic cross-sectional view, an implanting procedure for implanting the implant shown in Figs. 1 and 2;
- Fig. 6: shows, in a schematic cross-sectional view, an instrument for implanting an implant according to an embodiment of the present invention;
- Fig. 7: shows, in a schematic cross-sectional view, an instrument for implanting an implant according to a further embodiment of the present invention;
- Fig. 8: shows, in a schematic cross-sectional view, an instrument for implanting an implant according to a further embodiment of the present invention;
- Fig. 9: shows, in a schematic cross-sectional view, an instrument for implanting an implant according to a further embodiment of the present invention;
- Fig. 10: shows, in a schematic cross-sectional view, an instrument for implanting an implant according to a further embodiment of the present invention;
- Fig. 11: shows, in a schematic cross-sectional view, an instrument for implanting an implant according to a further embodiment of the present invention;
- Fig. 12A: shows, in a schematic cross-sectional view, an instrument for implanting an implant according to a further embodiment of the present invention;
- Fig. 12B: shows, in a schematic cross-sectional view, a modification of the instrument of Fig. 12A;
- Fig. 13: shows, in a schematic cross-sectional view, an instrument for implanting an implant according to a further embodiment of the present invention;
- Fig. 14: shows, in a schematic cross-sectional view, an instrument for implanting an implant according to a further embodiment of the present invention.

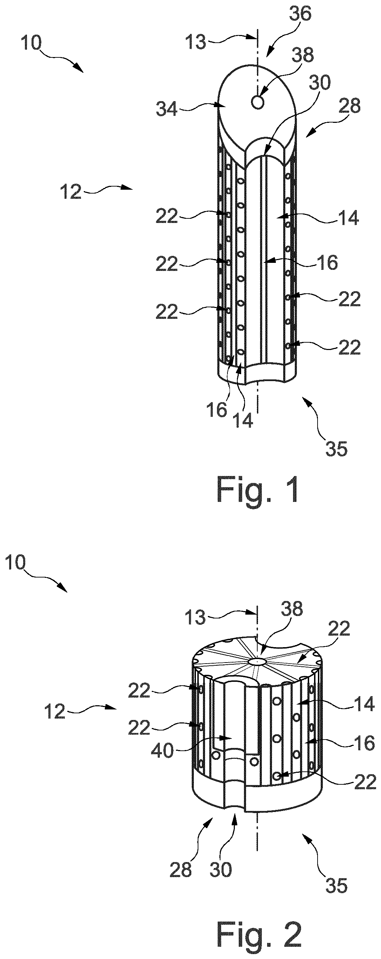

Fig. 1 shows, in a schematic perspective view, an implant 10 configured to be at least partially inserted into at least one opening in at least one bone of a patient. The implant 10 may include at least one implant body 12 made at least partially of at least one resorbable material. The implant 10, more specifically the implant body 12, may have a longitudinal axis 13.

The implant body 12 may include at least one first region 14 and at least one second region 16. The implant body 12 may include a plurality of first regions or first region segments 14 and/or a plurality of second regions or second region segments 16.

The first region(s) 14 may have a plurality of open pores. The first region(s) 14 may have a greater porosity than the second region(s) 16.

Preferably, each second region or second region segment 16 is separated from an adjacent second region or second region segment 16 by at least one of the first regions or first region segments 14, as shown in Fig. 1. Preferably, the second regions 16 are arranged in a network or framework manner, e.g., which at least partially encases the first regions 14.

The open pores of the first region(s) 14 may facilitate osseointegration or bone bridging of the implant 10 and the bone, e.g., by allowing bone ingrowth into the pores. This may accelerate osseointegration and reduce the risk of failure of the implant 10 and/or revision rates. For instance, the implant 10 may be configured to achieve substantially full osseointegration within 12 months after implanting the implant 10 into the patient.

The open pores of the first region(s) 14 may cause the first region 14 to be relatively brittle which is compensated by the increased stability and and/or strength which is provided by the denser second region(s) 16. In other words, the second region(s) 16 may protect the relatively highly osteoconductive, porous volume of the first region(s) 14. The second regions(s) 16 may provide a framework which at least partially encases the first region(s) 14.

The second region(s) 16 may provide a lattice-like support structure, i.e., a load-bearing framework, to the implant body 12 to maintain implant integrity while providing a relatively quick osseointegration of the implant 10 into the bone.

The first region(s) 14 may include a plurality of channels 22 defined at least partially in the first region(s) 14. Due to the relatively large number of channels 22 shown in the embodiment in Fig. 1, not each channel 22 is indicated by a reference sign in Fig. 1. The same applies to the first regions 14 and the second regions 16. The number of first regions 14, second regions 16, and channels 22 shown in Fig. 1 is only exemplary. The implant 10 may include fewer or more first regions 14, second regions 16, and/or channels 22 than shown in Fig. 1.

The channels 22 may have an open end which faces one or more walls of the bone, preferably one or more side walls of the bone, when the implant 10 is inserted in the opening of the bone.

The implant 10 may include at least one attachment means 28, e.g., for attaching connective tissue and/or a connective tissue replacement graft to the implant 10, preferably the implant body 12. The attachment means 28 may include at least one groove 30 which is defined in the implant body 12. The groove 30 may be configured to at least partially receive connective tissue and/or a connective tissue replacement graft. The groove 30 may extend at least partially along the longitudinal axis 13 of the implant body 12.

The implant 10 may include a distal end section 34, which terminates in a leading edge 36, and a proximal end section 35. The distal end section 34 may be tapered towards the leading edge 36. The distal end section 34 and/or the proximal end section 35 may be at least partially formed by the first region(s) 14.

The implant 10 may include at least one through-opening 38 which may extend at least partially through the implant body 12, preferably along the longitudinal axis 13. The through-opening 38 may be configured to interact with, preferably to receive, at least a portion of an instrument or tool for at least partially assisting in implanting the implant 10.

Fig. 2 shows, in a schematic cross-sectional view, an implant 10 according to a further embodiment of the present invention. The embodiments of Figs. 1 and 2 may be combined or may be practiced separately.

As shown in Fig. 2, the implant 10 may further include at least one securing device 40 configured to secure the implant 10 to the bone, at least until osseointegration or bone bridging between the implant 10 and the bone has occurred. The implant 10 may include a plurality of the at least one securing device 40.

The securing device 40 may be configured to interact with the bone, preferably by a form-fitting connection and/or a frictional connection, to fixate the implant 10 to the bone, at least until osseointegration or bone bridging between the implant 10 and the bone has occurred, and optionally also after osseointegration or bone bridging between the implant 10 and the bone has occurred.

The securing device 40 may be configured as an insert, e.g., a pin, which is at least partially attached to the implant body 12. The securing device 40 may be at least partially made of a relatively dense material, e.g., the securing device 40 may have a greater density than the implant body 12.

The securing device 40 may be made at least partially of an at least partially modifiable, preferably liquifiable, material. Preferably, the securing device 40 is made at least partially of an at least partially liquifiable material which is liquifiable in a state in which the implant 10, or at least the securing device 40, is at least partially inserted in the opening of the bone, preferably by applying energy, preferably ultrasound energy, to at least a portion of the securing device 40.

The securing device 40 may be made at least partially of an at least partially curable and/or hardenable material which is curable in a state in which the implant 10 or at least the securing device 40 is inserted in the opening of the bone. The curable and/or hardenable material may be passively curable and/or hardenable, e.g., by stopping an application of energy to the securing device 40, e.g., by letting the securing device 40 passively cool and harden and/or solidify. Additionally, or alternatively, the curable and/or hardenable material may be actively curable and/or hardenable, e.g., by actively cooling and/or applying pressure to the securing device 40.

To implant the implant 10 in a patient's body, the implant 10 may be inserted at least partially into at least one opening in at least one bone of the patient.

Connective tissue, such as a ligament or tendon, and/or a connective tissue replacement graft, such as a ligament graft and/or a tendon graft, may be attached to the implant 10, preferably the implant body 12, e.g., via the attachment means 28.

The implant 10 may be fixated to the bone, e.g., by at least partially liquifying the liquifiable material of the securing device 40. The securing device 40 may be pre-attached to the implant body 12 or may be inserted into the implant body 12 and/or into the opening in the bone, once the implant body 12 has been at least partially inserted into the opening in the bone and/or may be inserted into the opening in the bone simultaneously with the implant body 12.

The above-described means of fixating the implant 10 is only exemplary and may be the preferred fixation means. However, a number of further fixation means may be employed. For instance, the securing device 40 may be made at least partially of a modifiable material, which is modifiable, preferably in a state in which the implant, or at least the securing device, is at least partially inserted in the opening of the bone, by adjusting at least one of at least one shape, at least one dimension, an aggregate state, a consistency, a plasticity, a viscosity and a hardness of the modifiable material. A fixation between the securing device 40 and the bone may be achieved, or at least triggered, by modifying the modifiable material.

In particular, modifying, e.g., liquifying, the modifiable material may trigger and/or change an interaction, e.g., a form-fitting connection and/or a frictional connection, between the modifiable material and the bone to fixate the implant 10 to the bone, and optionally also between the modifiable material and connective tissue and/or at least one connective tissue replacement graft to fixate the connective tissue and/or at least one connective tissue replacement graft to the bone.

The securing device 40 may be at least partially modified, e.g., liquified, by applying energy, preferably ultrasound energy and/or thermal energy, to at least a portion of the securing device 40. The energy may be applied directly from a source of energy, e.g., a source of thermal energy and/or a source of ultrasound energy, directly or indirectly to the securing device 40, e.g., by contacting the securing device 40 directly with the source of energy, e.g., via the groove 30 or by applying the energy to the implant body 12.

Optionally, the implant 10 may be fixated to at least one second bone.

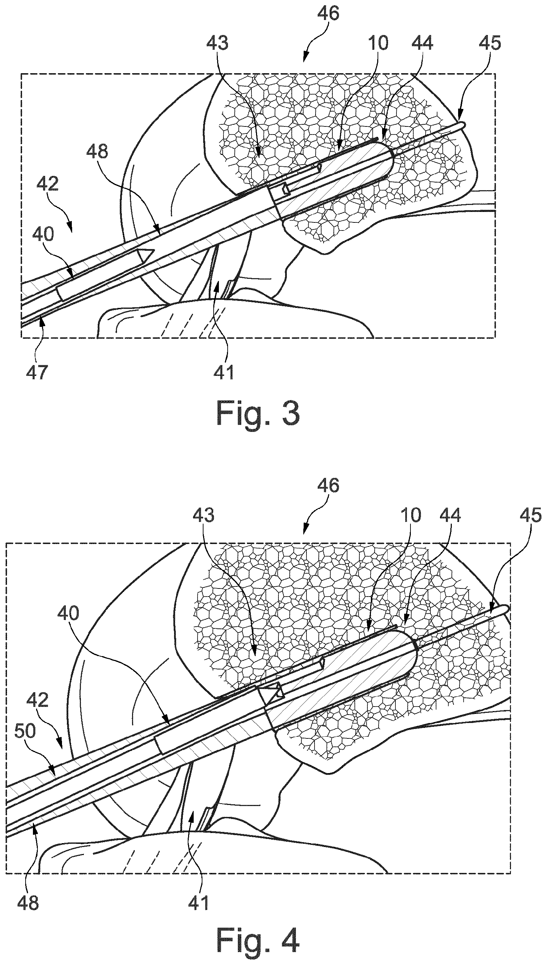

Figs. 3 to 5 show different stages of an implanting procedure for implanting the implant 10 into a patient's body with the aid of an instrument 42. Figs. 3 to 5 show, in an exemplary manner, a procedure for attaching a graft 41 to a bone 46. However, a range of further applications are feasible for employing the implant 10 and/or the instrument 42, e.g., for conversion osteotomies, for connecting/bridging resected joints in arthrodesis, in arthrodesis systems, e.g., in foot and ankle, and/or for intramedullary fixation, e.g., in foot and ankle.

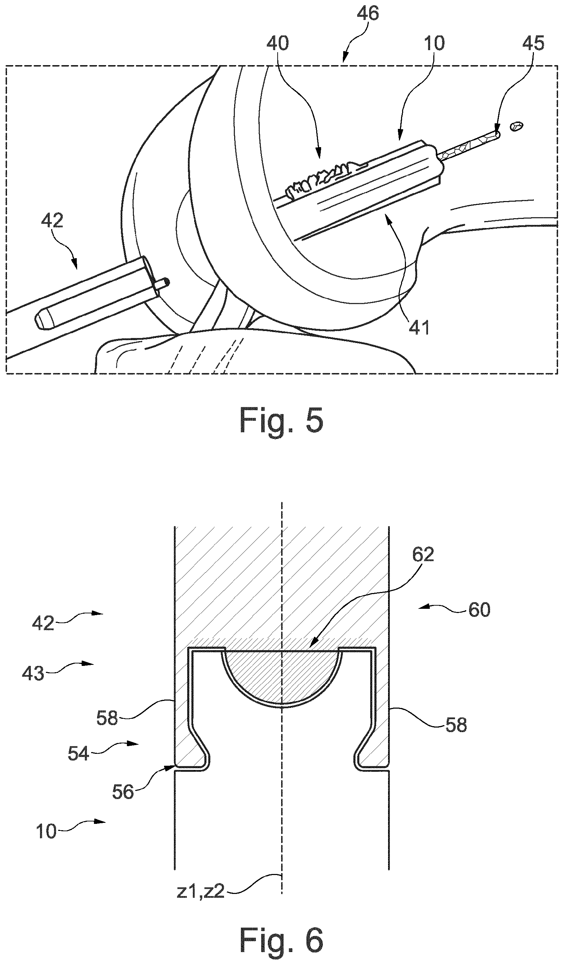

As shown in Fig. 3, the implant 10 may be fixedly attached to the instrument 42, preferably a distal end 43 of the instrument 42. Various exemplary attachment mechanisms for attaching the implant 10 to the instrument 42 are shown in Figs. 6 to 14 and described below in relation to said Figs. 6 to 14. With the implant 10 firmly attached to the instrument 42, the implant 10 may be inserted into at least one opening 44 in the bone 46 of the patient. At least one fixating device 45, e.g., at least one k-wire, may be employed to temporarily fixate the instrument 42 and/or the implant 10 and/or the graft 41 to the bone 46. Once the implant 10 is in place within the opening 44, the securing device 40, which may be configured as a pin as shown in Figs. 3 to 5, may be displaced through a lumen or channel 48 defined within the instrument 42 to the implant 10. For this, a maneuvering device 47 configured to be inserted into the lumen or channel 48 and to maneuver, e.g., push, the securing device 40 towards the implant 10 may be provided. Fig. 4 shows the securing device 40 in a position adjacent to the implant 10. Alternatively, the securing device 40 may be pre-mounted to and/or in the implant 10, e.g., in a cavity defined within the implant 10, prior to inserting the implant 10 into the opening 44 of the bone 46.

In the position of the securing device 40 shown in Fig. 4, an energy applicator 50, e.g., a sonotrode, may be introduced through the channel 48 such that a distal end of the energy applicator 50 may engage with the securing device 40. The energy applicator 50 may apply energy, e.g., thermal energy and/or a source of ultrasound energy, to the securing device 40, thereby at least partially altering, e.g., liquifying, the securing device 40. As discussed above, this may trigger and/or change an interaction, e.g., a form-fitting connection and/or a frictional connection, between the securing device 40 and the bone 46 to fixate the implant 10 to the bone 46, and optionally also between the securing device 40 and the graft 41 to fixate the graft 41 the bone 46. The maneuvering device 47 and the energy applicator 50 may be the same device. However, alternatively, the maneuvering device 47 and the energy applicator 50 may be separate devices.

As shown in Fig. 5, the modified/liquefied material of the securing device 40 may engage with, e.g., flow and/or be pressed at least partially into, the bone 46 and the implant 10, whereupon the material of the securing device 40 may solidify to fixedly connect the implant 10 to the bone 46.

As detailed above, Figs. 6 to 14 show various exemplary attachment mechanisms for attaching the implant 10 to the instrument 42. For instance, as shown in Fig. 6, the instrument 42 may be configured such that the implant 10 is attachable to and/or detachable from the instrument 42 by translationally displacing the instrument 42 relative to the implant 10 and engaging the instrument 42, preferably the distal end 43 of the instrument 42, with the implant 10. In other words, the instrument 42 may be attached to the implant 10 by merely pushing the instrument 42 against the implant 10 and/or vice versa, thereby actuating or activating one or more engagement mechanisms for fixedly attaching the implant 10 to the instrument 42. To detach the instrument 42 from the implant 10, the instrument 42 may merely be pulled, e.g., proximally, from the implant 10, when the implant 10 has been implanted within the patient's body, to release the one or more engagement mechanisms. To achieve such a non-rotating-based connection, e.g., a screw connection, the instrument 42 may include at least one retention device 54 configured to engage with and/or be received in at least one undercut 56 defined in at least a section of the implant 10 to releasably fix the implant 10 to the instrument 42. The retention device 54 may be arranged at the distal end 43 of the instrument 42.

The retention device 54 may be at least partially deflectable, preferably in a direction which is substantially perpendicular to a longitudinal axis z1 of the instrument 42 and/or a longitudinal axis z2 of the implant 10, such that the retention device 54 is at least partially deflected, when the implant 10 is being attached to and/or detached from the instrument 42. In particular, as shown in Fig. 6, the retention device 54 may be configured as or may include one or more deflectable arms 58 which extend distally from a main section 60 of the instrument 42. The deflectable arms 58 may be configured to engage with the implant 10 via a snap fit. The retention device 54, e.g., the deflectable arms 58, may be configured such that the implant 10 is released from the instrument 42 merely by displacing the instrument 42 proximally, e.g., by merely pulling the instrument 42 from the implant 10. In other words, a distal force may not need to be exerted onto the implant 10 by the instrument 42, or a designated release device, in order to release the implant 10 from the instrument 42. In particular, one of more forces holding the implant 10 in place in the opening 44 of the bone 46, e.g., friction between the implant 10 and the bone 46, may be greater than a connecting force between the retention device 54 and the implant 10.

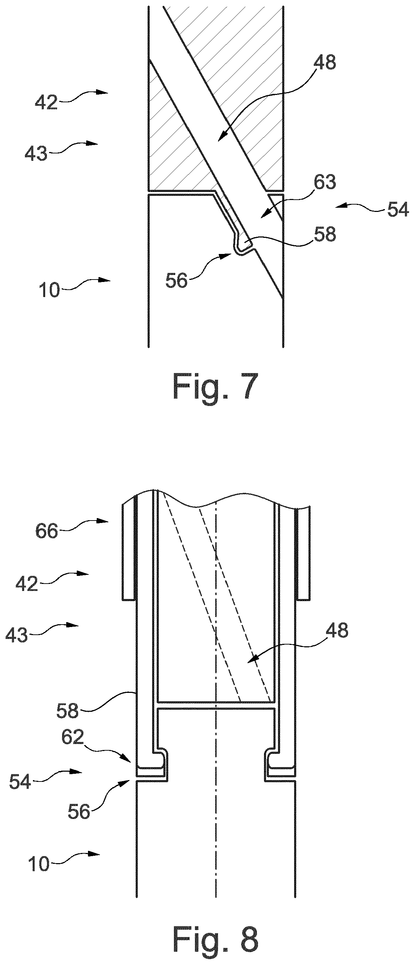

The instrument 42 may include at least one protective member 62 arranged at least partially at an interface between the instrument 42 and the implant 10. The protective member 62 may be configured to absorb one or more loads and/or shocks between the instrument 42 and the implant 10. In other words, the protective member 62 may be configured to absorb, and preferably dampen, any forces, loads, and/or shocks which may occur between the instrument 42 and the implant 10. This may be achieved by manufacturing the protective member 62 from a suitable material, e.g., a material which is more compressible and/or softer and/or has a greater degree of internal damping than an adjacent material of the instrument 42 and/or the implant 10, and/or by configuring the shape of the protective member 62 accordingly, e.g., as a spring-like element. The retention device 54 may include the protective member 62. Alternatively, or additionally, the protective member 62 may be arranged at a distal end of the deflectable arms 58 (see Fig. 8).

Fig. 7 shows a modification of the embodiment of Fig. 6. In particular, the retention device 54, e.g., at least one deflectable arm 58 thereof, may be configured to engage an undercut or groove 56 arranged within a cavity or channel 63 of the implant 10. The cavity or channel 63 may be provided in the implant 10 for one or more other functions, e.g., for introducing the securing device 40 shown in Figs. 1 to 5 and described above. Hence, the cavity/channel 63 may already be present in the implant 10 for other reasons and may be utilized for attaching the implant 10 to the instrument 42 as described above.

According to the embodiment of Fig. 8, the instrument 42 may include at least one sheath 66 which is at least partially retractable relative to the implant 10, preferably in a proximal direction. The sheath 66 may be configured to at least partially circumferentially cover the retention device 54, e.g., the deflectable arm(s) 58, and/or the implant 10 at least in a first position of the sheath 66. The sheath 66 may be configured to be at least partially retracted from the first position to a second position to at least partially uncover or further uncover the retention device 54 and/or the implant 10.

The sheath 66, when in the first position, may prevent the implant 10 from releasing from the instrument 42, or at least may reduce the risk thereof. For instance, the sheath 66 may be configured to exert a force, e.g., a radial force, onto the arms 58 of the retention device 54, thereby urging the arms 58 towards the implant 10 or at least preventing the arms 58 from being deflected outwards, i.e., away from the implant 10, or at least reducing the risk thereof. Retracting the sheath 66 to the second position may allow the retention device 54 to be released from the implant 10 which may allow the release of the implant 10 from the instrument 54.

The sheath 66 may include one or more openings and/or slots, e.g., to allow the securing device 40 to be passed and/or to flow therethrough to an interface between the implant 10 and the bone 46 and/or to facilitate retraction of the sheath 66 over the securing device 40.

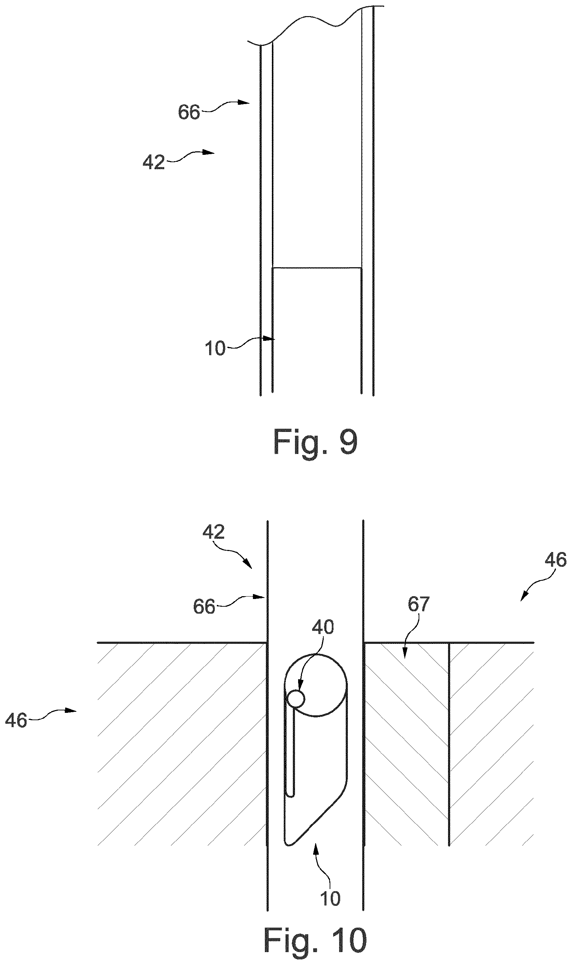

As shown in Fig. 8, protective member 62 may be arranged along a section of the arms 58, in particular at a distal end of the arms 58. Fig. 9 shows a modification of the embodiment of Fig. 8. As shown in Fig. 9, the sheath 66 may be employed without the retention device 54 shown in Fig. 8. In this case, the sheath 66 may engage directly with the implant 10, e.g., by at least partially, preferably completely, extending distally along a length of the implant 10, to fixate the implant 10 with the sheath 66. The implant 10 may be released from the instrument 42 by at least partially retracting the sheath 66 in a proximal direction to at least partially uncover or further uncover the implant 10.

Fig. 10 shows a further modification of the embodiment of Fig. 8. In particular, as shown in Fig. 10, the sheath 66 may be configured to exert at least one force, preferably at least one force which is directed radially outward, onto the bone 46. Since a portion of the bone 46, e.g., the spongy bone tissue 67, is relatively soft, the force exerted by the sheath 66 onto the bone 46 may elastically urge/push the surrounding or adjacent bone tissue 67 away, preferably radially outward, from the implant 10. Retracting the sheath 66 proximally may allow the bone tissue 67 to at least partially return to its original state, i.e., towards the implant 10. The implant 10 may be sized and/or shaped such that, when the sheath 66 has been retracted, the bone tissue 67 engages with and presses again the implant 10, to effectively form a frictional/press fit between the bone 46 and the implant 10 to substantially hold the implant 10 in place.

The sheath 66 may include one or more openings and/or slots, e.g., to allow the securing device 40 to be passed and/or to flow therethrough to an interface between the implant 10 and the bone 46 and/or to facilitate retraction of the sheath 66 over the securing device 40.

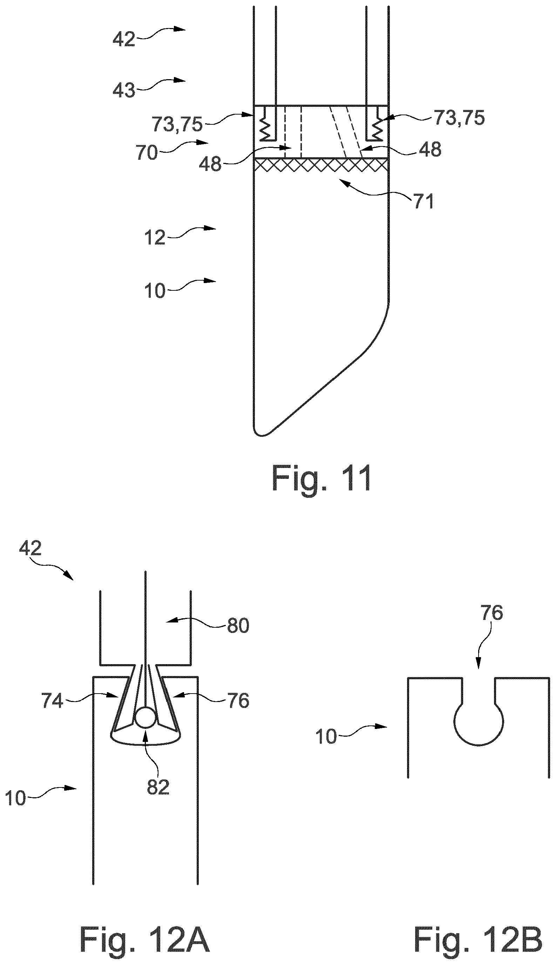

As shown in Fig. 11, at least one adapter 70 may be provided to facilitate attachment of the implant 10 to the instrument 42. The adapter 70 may be made of a different material than the implant body, e.g., the implant body 12, of the implant 10. The adapter 70 may be configured to be engaged by an instrument 42 for implanting the implant 10.

Hence, the adapter 70 may be provided as an intermediate member between the instrument 42 and the main body 12 of the implant 10. In other words, the adapter 70 may provide an interface between the instrument 42 and the main body 12. This may allow the adapter 70 to be configured to facilitate an attachment of the implant 10 to the instrument 42, i.e., without potentially causing adverse effects to the main body 12 of the implant body 10, or at least reducing the adverse effects. This may allow the configuration, e.g., the material and/or the porosity, of the main body 12 to be targeted more specifically to osteointegration and/or bone bridging between the implant 10 and the bone 46, whereas the function of connecting the implant 10 to the instrument 42 may be primarily provided and/or facilitated by the adapter 70.

The adapter 70 may be fixedly attached, at an attachment interface 71, to the main body 12, preferably such that the adapter 70 remains attached to the main body 12, after the implant 10 has been released by the instrument 42. The adapter 70 may be adhesively bonded to the main body 12, e.g., using the same principle and/or the same material as the material described above for connecting the implant 10 with the bone 46. The adapter 70 may be made of a biocompatible material. For instance, the adapter 70 may be made of polyether ether ketone (PEEK) and/or polylactic acid (PLA) and/ or a biocompatible elastomer, e.g., a polycarbonate urethane (PCU).

The adapter 70 may be include one or more screw threads 73 configured to engage with one or more screw threads 75 defined in or on the instrument 42. In other words, the adapter 70 may allow a screw connection to be employed for connecting the instrument 42 to the implant 10, via the adapter 70, without limiting, or at least reducing limitations of, the choice of material of the main body 12 of the implant 10. Thus, this may allow a broader range of materials, in which screw threads may not be provided or in which screw threads are less suitable and/or disadvantageous, e.g., materials with a higher porosity, to be used for manufacturing the main body 12 of the implant 10.

As shown in Figs. 12A and 12B, the instrument 42 may include at least one expandable member 74 configured to be inserted into at least one cavity 76 of the implant 10. The expandable member 74 may be configured to expand once the expandable member 74 has been inserted in the cavity 76. A tensioning member 80 may be included for providing an additional expanding force onto the expandable member 74, i.e., to increase the connective forces between the instrument 42 and the implant 10. In particular, the tensioning member 80 may exert a proximal force onto the expandable member 74, e.g., by pulling the tensioning member 80 in a proximal direction. The tensioning member 80 may also provide (axial) tension between the instrument 42 and the implant 10, e.g., to maintain the implant 10 in a substantially stationary position relative to the instrument 42. This may reduce potential movement, in particular axial movement, of the implant 10 relative to the instrument 42. This may increase the accuracy and/or efficiency of the implanting procedure of the implant 10 into the patient's body. For instance, the tensioning member 80 may be configured as a band, preferably a stretchable band, or thread. The tensioning member 80 may have an enlarged distal end 82 to facilitate an engagement between the tensioning member 80 and the expandable member 74.

As shown in Fig. 12A, the cavity 76 may be substantially conically shaped and/or tapered in a proximal direction. Alternatively, the cavity 76 may have a greater radius at least in a section thereof. For instance, at least a section of the cavity 76 may be substantially circular and/or oval. The size and/or shape of the expandable member 74 may be adapted to the size and/or shape of the cavity 76 accordingly.

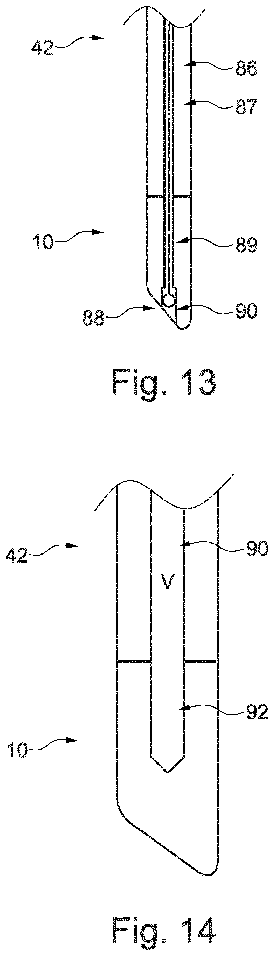

As shown in Fig. 13, the instrument 42 may include at least one tensioning member 86 configured to exert at least one force onto the implant 10 in a proximal direction. The tensioning member 86 may extend through a channel 87 of the instrument 42 and a channel 89 of the implant 10. The tensioning member 86 may have an enlarged distal end 88 configured to engage in a cavity 90 of the implant 10 to transmit the tensioning force onto the implant 10.

As shown in Fig. 14, the instrument 42 may be configured to generate a vacuum V to fix the implant 10 to the instrument 42. For instance, the instrument 42 may include at least one vacuum generating mechanism (not shown), e.g., a pump, for generating the vacuum. The vacuum generating mechanism may be manually driven, e.g., as a hand-driven pump, and/or may be electrically and/or pneumatically powered. The vacuum V may be generated in at least one cavity 90 defined within the instrument 42. The cavity 90 of the instrument 42 may be operatively connected to the implant 10, e.g., to one of more surfaces and/or cavities of the implant 10, to provide a vacuum-based attachment force between the instrument 42 and the implant 10 to fixedly connect the instrument 42 to the implant 10, at least until the vacuum V is reduced and/or ceases to prevail. A cavity 92 defined within the implant 10 may be fluidically connected to the cavity 90 of the instrument 42. Hence, this may result in the vacuum V also prevailing in the cavity 92 defined within the implant 10, which may increase the attachment force between the instrument 42 and the implant 10.

## Claims

1. An implant (10) configured to be at least partially inserted into at least one opening (44) in at least one bone (46) of a patient, the implant (10) including:
at least one implant body (12) made at least partially of at least one resorbable material;
wherein the implant body (12) includes at least one first region (14) and at least one second region (16),
wherein the first region (14) has a plurality of open pores; and
wherein the first region (14) has a greater porosity than the second region (16).

2. The implant (10) according to claim 1, wherein the implant (10) is configured to:
connect the bone (46) to at least one further structure of the patient's body, preferably at least one second bone;
and/or
fixate at least one connective tissue (41) and/or at least one connective tissue replacement graft (41) to the bone (46), when the implant (10) is at least partially inserted into the opening (44) of the bone (46).

3. The implant (10) according to claim 1 or 2, wherein the implant (10) is configured to transfer one or more loads:
between the bone (46) and connective tissue (41) and/or between the bone (46) and at least one connective tissue replacement graft (41);
and/or
between the bone (46) and at least one further structure of the patient's body, preferably at least one second bone.

4. The implant (10) according to claim 2 or 3, wherein:
the connective tissue is a ligament, preferably an anterior cruciate ligament, and/or a tendon;
and/or
the connective tissue replacement graft (41) is a ligament graft and/or a tendon graft, preferably a graft for at least partially reconstructing an anterior cruciate ligament.

5. The implant (10) according to any of the preceding claims, wherein the first region (14) includes a plurality of channels (22) defined at least partially in the first region (14), preferably wherein the channels (22) have an open end which faces one or more walls of the bone (46), preferably one or more side walls of the bone (46), when the implant (10) is inserted in the opening (44) of the bone (46).

6. The implant (10) according to any of the preceding claims, further including at least one securing device (40) configured to interact with the bone (46), preferably by a form-fitting connection and/or a frictional connection, to fixate the implant (10) to the bone (46), wherein the securing device (40) is made at least partially of an at least partially liquifiable material which is liquifiable in a state in which the implant (10), or at least the securing device (40), is at least partially inserted in the opening (44) of the bone (46), preferably by applying energy, preferably ultrasound energy, to at least a portion of the securing device (40).

7. The implant (10) according to any of the preceding claims, including a plurality of the at least one first region (14) and a plurality of the at least one second region (16), wherein each second region (16) is separated from an adjacent second region (16) by at least one of the first regions (14).

8. The implant (10) according to claim 7, wherein at least some of the first regions (14) and/or at least some of the second regions (16) extend axially along the implant body (12).

9. The implant (10) according to any of the preceding claims, further including at least one adapter (70) made of a different material than the implant body (12), wherein the adapter (70) is configured to be engaged by an instrument (42) for implanting the implant (10).

10. An instrument (42) for implanting an implant (10) according to any of the preceding claims into the at least one opening (44) defined in at least one bone (46) of a patient.

11. The instrument (42) according to claim 10, wherein the instrument (42) is configured such that the implant (10) is attachable to and/or detachable from the instrument (42) by translationally displacing the instrument (42) relative to the implant (10) and engaging the instrument (42), preferably a distal end of the instrument (42), with the implant (10).

12. The instrument (42) according to claim 10 or 11, wherein the instrument (42) includes at least one retention device (54) configured to engage with and/or be received in at least one undercut (56) defined in at least a section of the implant (10) to releasably fix the implant (10) to the instrument (42), preferably wherein the retention device (54) is arranged at a distal end (43) of the instrument (42).

13. The instrument (42) according to any of claims 10 to 12, wherein the instrument (42) includes at least one protective member (62) arranged at least partially at an interface between the instrument (42) and the implant (10), wherein the protective member (62) is configured to absorb one or more loads and/or shocks between the instrument (42) and the implant (10).

14. A kit including an implant (10) according to any of claims 1 to 9 and an instrument (42) according to any of claims 10 to 13.

15. A method for implanting at least one implant (10) in at least one opening (44) in at least one bone (46) of a patient, preferably using an instrument (42) according to any of claims 10 to 13, the method including:
(a) providing the implant (10) which includes at least one implant body (12) made at least partially of at least one resorbable material, wherein the implant body (12) includes at least one first region (14) and at least one second region (16), wherein the first region (14) has a plurality of open pores; and wherein the first region (14) has a greater porosity than the second region (16);
(b) inserting the implant (10) at least partially into the opening (44) in the bone (46); and
(c) fixating the implant (10) to the bone (46).
